# EUROPEAN PATENT APPLICATION

(11) **EP 2 476 903 A1**
(43) Date of publication of application: **18.07.2012**
(21) Application number: 11194891.5
(22) Date of filing: 21.12.2011
(51) Int. Cl.: F04B 43/12

(54) **Peristaltic pumps and filtration assembly systems for use therewith**

(30) Priority: 13.01.2011 US 201113005603
(71) Applicant: Pall Corporation, Port Washington, NY 11050 (US)
(72) Inventor: Steere, William Charles, Grass Lake, MI Michigan 49240 (US); McClain, Michael Scott, Waterford, MI Michigan 48329 (US); Bormann, Thomas J., Huntington, NY New York 11743 (US)
(74) Representative: Hoeger, Stellrecht & Partner Patentanwälte

(57) **Abstract**

Filtration assemblies for use with peristaltic pumps, peristaltic pumps, systems including the assemblies and pumps, and methods of using the assemblies, pumps, and systems, are disclosed.

## Description

### BACKGROUND OF THE INVENTION

The Membrane Filtration Technique is an internationally recognized reference technique for microbial analysis. In accordance with the technique, a fluid sample potentially containing microbial contamination is passed through a collection membrane (typically arranged in a filter device), and microbes remaining with the membrane are exposed to liquid nutritive media to allow the microbes to grow and replicate. Subsequently, the membrane or nutritive fluid is analyzed to determine the presence of microbes.

Systems for use with conventional microbial filters typically utilize a vacuum pump downstream of the membrane to provide for passing the fluid sample through the membrane, and the system includes a collection reservoir between the membrane and the vacuum pump to collect fluid to prevent the fluid from entering the vacuum pump. Such systems should be regularly cleaned and sanitized to avoid contamination, as that contamination can lead to contamination of the fluid sample to be analyzed. Other systems for use with microbial filters utilize an internal liquid pump to provide for passing the fluid sample through the membrane. The fluid sample and liquid nutritive media come in contact with internal components of the pump, and as a result, the pump must also be sanitized.

There is a need for improved devices, systems, and methods for carrying out the membrane filtration technique for microbial analysis. These and other advantages of the present invention will be apparent from the description as set forth below.

### BRIEF SUMMARY OF THE INVENTION

An embodiment of the invention provides a filtration assembly system for use with a peristaltic pump comprising a filtration assembly, the assembly comprising a sample reservoir for holding a fluid sample to be filtered; a fluid port in fluid communication with the sample reservoir; a filter element disposed in a flow path between the sample reservoir and the fluid port; a filter support surface for supporting the filter element; wherein the system further comprises a flexible tube having a first end and a second end, wherein the first end is connectable to the fluid port to provide fluid communication between the first end and the port, or the first end is connected to the fluid port and the first end is in fluid communication with the port. In some embodiments of the filtration assembly system, the filtration assembly further comprises a cover member detachably and replaceably covering the sample reservoir with a fluid-tight fit and/or a base supporting the sample reservoir. In preferred embodiments of the filtration assembly system, the filtration assembly further comprises the base, wherein the base comprises the fluid port and/or the base comprises the filter support surface for supporting the filter element.

In another embodiment, a peristaltic pump for use with a filtration assembly system is provided, comprising a peristaltic pump head, the head comprising a head housing, a rotatable rotor comprising at least two shoes or rollers, and a tubing holder suitable for receiving flexible tubing, the flexible tubing having an outside diameter, wherein the shoes or rollers are arranged to compress the flexible tubing placed in the holder, and wherein the flexible tubing is in fluid communication with a fluid port of a disposable filtration assembly; the fluid port, having an outside diameter, also being in fluid communication with a sample reservoir of the disposable filtration assembly, the fluid port being arranged to receive filtered liquid passing through a filter element in the filtration assembly; a motor communicating with the pump head, arranged to rotate the rotor; a pump housing receiving the motor, the pump housing comprising a pump housing cover, the cover including (i) a substantially planar filtration assembly receiving portion and (ii) a slot in the receiving portion, wherein the slot includes a gap at least as large as the outer diameter of the fluid port of the disposable filtration assembly, wherein the gap also is as large as the outer diameter of the flexible tubing without significantly compressing the tubing; wherein the pump is arranged to pull fluid in a flow direction through the sample reservoir, the filter element, the fluid port, and the flexible tubing, without allowing fluid flow in the opposite direction.

A method for processing a fluid potentially containing microbial contaminants according to an embodiment of the invention comprises operating a peristaltic pump downstream of, and in fluid communication via flexible tubing with, a filtration assembly comprising a sample reservoir, a filter element, and a fluid port, the sample reservoir containing the fluid potentially containing microbial contaminants; wherein operating the pump comprises compressing and opening the flexible tubing and pulling the fluid in a flow direction through the sample reservoir, the filter element, the fluid port, and the flexible tubing, without allowing fluid flow in the opposite direction.

### BRIEF DESCRIPTION OF THE SEVERAL VIEWS OF THE DRAWING(S)

Figure 1 shows an embodiment of a peristaltic pump according to the present invention. Figure 1A is an exploded view of the peristaltic pump, showing a pump head engaged with a motor, and a pump housing including a slot for receiving the fluid port of a filtration assembly and/or for receiving an adapter for attaching to the fluid port. Figure 1B is a perspective view of an assembled pump.

Figure 2 shows an illustrative pump head, Figure 2A shows the pump head when closed and Figure 2B shows the pump head when open.

Figure 3 shows an embodiment of a filtration assembly system (comprising a filtration assembly and an attached tubing adapter) according to the invention engaged with an embodiment of a peristaltic pump according to the invention. Figure 3A shows a perspective view, and Figure 3B shows a cross-sectional view.

Figure 4 shows perspective views of two illustrative embodiments of filtration assembly systems according to the invention, each comprising a filtration assembly and attachable tubing.

Figure 5 shows an isometric view of an exemplary filtration assembly for use in the invention.

Figure 6 is a vertical cross-sectional view of the filtration assembly shown in Figure 5.

Figure 7 is a top isometric view of the exemplary base shown in Figure 5.

Figure 8 shows an embodiment of an adapter for attaching to a fluid port of a filtration assembly, wherein flexible tubing is attached to the adapter, wherein the adapter and tubing are sealed in a package.

### DETAILED DESCRIPTION OF THE INVENTION

Advantageously, in contrast with conventional systems including a vacuum pump or an internal liquid pump, the invention provides for a reduced number of connections between components, each of which is a potential point of breach wherein microbial contamination (e.g., leading to contamination of the fluid to be sampled) may occur. Thus, the fluid pathway is simplified.

Another advantage is that the all of the components wetted by fluid in accordance with the invention, i.e., the filtration assembly, and more importantly, the tubing adapter (if present) and the tubing, can be easily removed, without risk of contamination of the peristaltic pump, as the peristaltic pump is not wetted by fluid. In contrast, the components wetted by (or potentially wetted by) fluid in conventional systems include a vacuum pump or an internal liquid pump, which increases the risk of contamination carry over, requiring separate sanitation procedures, such as autoclave sanitization (vacuum systems) or pumping sanitization fluid through the system (internal liquid pump systems).

Alternatively, or additionally, other advantages include one or more of the following: (a) the tubing/tubing adapter can be supplied pre-sterilized in a package, requiring no sanitation procedure between tests; (b) fewer fluid contacting components are needed downstream of the filtration assembly, e.g., the invention can be carried out using a short length of tubing (with or without an adapter), whereas conventional systems include, for example, a manifold assembly, a flask, a vacuum pump, a filter between the flask and the vacuum pump, and separate tubing connecting the manifold assembly to the flask, the flask to the filter, and the filter to the vacuum pump; (c) the peristaltic pump prevents any backflow onto the collection filter in the filtration assembly; and (d) the invention can be carried out while minimizing the footprint as compared to conventional systems, which is particularly desirable in some environments, e.g., in a positive pressure hood, where space is at a premium.

An embodiment of the invention provides a filtration assembly system for use with a peristaltic pump comprising a filtration assembly, the assembly comprising a sample reservoir for holding a fluid sample to be filtered; an optional cover member detachably and replaceably covering the sample reservoir with a fluid-tight fit; a fluid port in fluid communication with the sample reservoir; a filter element disposed in a flow path between the sample reservoir and the fluid port; and a filter support surface for supporting the filter element; wherein the system further comprises a flexible tube having a first end and a second end, wherein the first end is connectable to the fluid port to provide fluid communication between the first end and the port, or the first end is connected to the fluid port and the first end is in fluid communication with the port. In some embodiments of the filtration assembly system, the filtration assembly further comprises a base supporting the sample reservoir. In preferred embodiments of the filtration assembly system, the filtration assembly comprises the base, wherein the base comprises the fluid port and/or the base comprises the filter support surface for supporting the filter element.

In another embodiment, a peristaltic pump for use with a filtration assembly system is provided, comprising a peristaltic pump head, the head comprising a head housing, a rotatable rotor comprising at least two shoes or rollers, and a tubing holder suitable for receiving flexible tubing, the flexible tubing having an outside diameter, wherein the shoes or rollers are arranged to compress the flexible tubing placed in the holder, and wherein the flexible tubing is in fluid communication with a fluid port of a disposable filtration assembly; the fluid port, having an outside diameter, also being in fluid communication with a sample reservoir of the disposable filtration assembly, the fluid port being arranged to receive filtered liquid passing through a filter element in the filtration assembly; a motor communicating with the pump head, arranged to rotate the rotor; a pump housing receiving the motor, the pump housing comprising a pump housing cover, the cover including (i) a substantially planar filtration assembly receiving portion and (ii) a slot in the receiving portion, wherein the slot includes a gap at least as large as the outer diameter of the fluid port of the disposable filtration assembly, wherein the gap also is as large as the outer diameter of the flexible tubing without significantly compressing the tubing; wherein the pump is arranged to pull fluid in a flow direction through the sample reservoir, the filter element, the fluid port, and the flexible tubing, without allowing fluid flow in the opposite direction.

A method for processing a fluid potentially containing microbial contaminants according to an embodiment of the invention comprises operating a peristaltic pump downstream of, and in fluid communication via flexible tubing with, a filtration assembly comprising a sample reservoir, a microbe-collecting filter element, and a fluid port, the sample reservoir containing the fluid potentially containing microbial contaminants; wherein operating the pump comprises compressing and opening the flexible tubing and pulling the fluid in a flow direction through the sample reservoir, the filter element, the fluid port, and the flexible tubing, without allowing fluid flow in the opposite direction.

Each of the components of the invention will now be described in more detail below, wherein like components have like reference numbers.

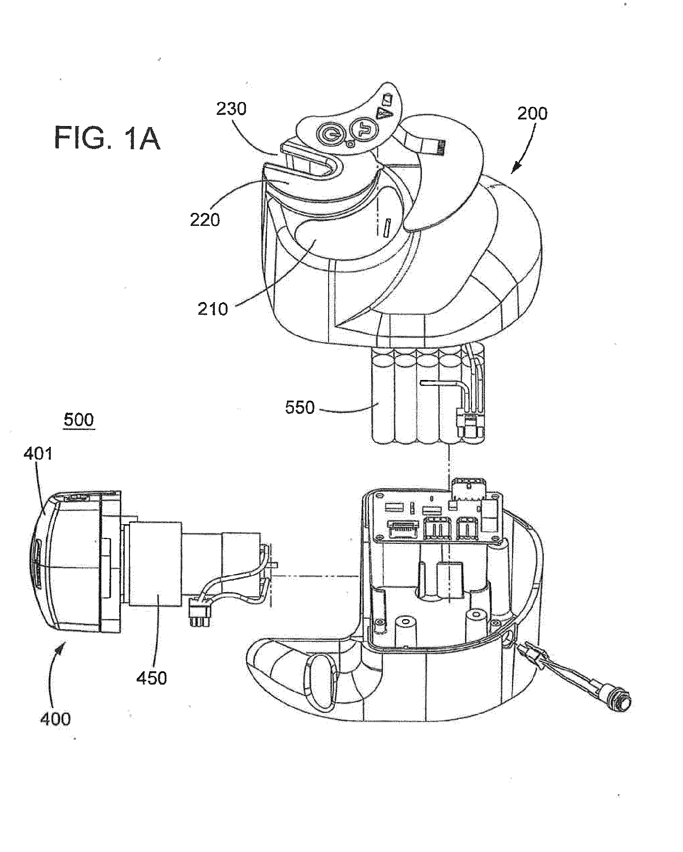

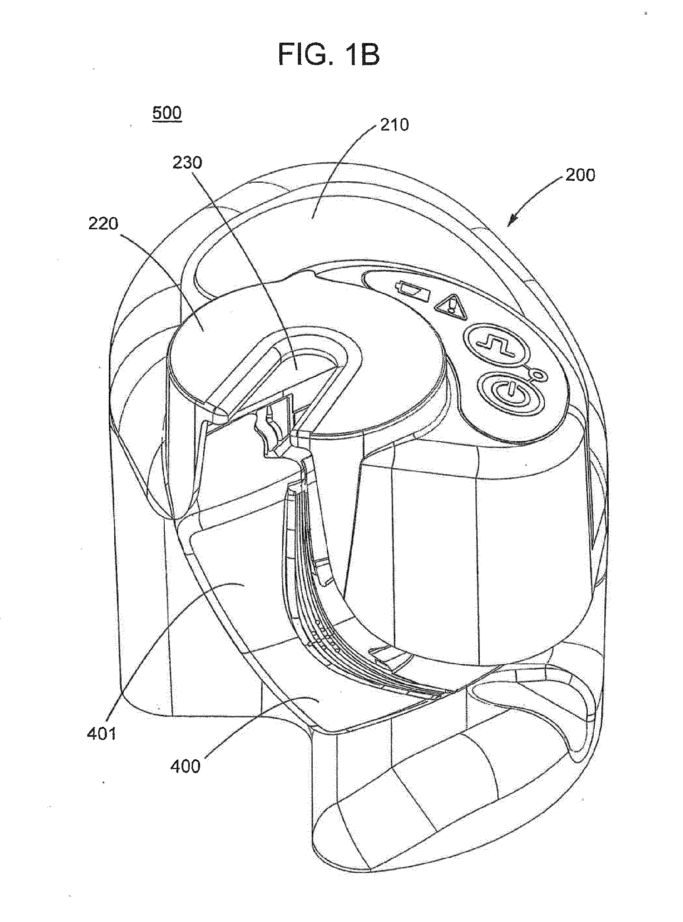

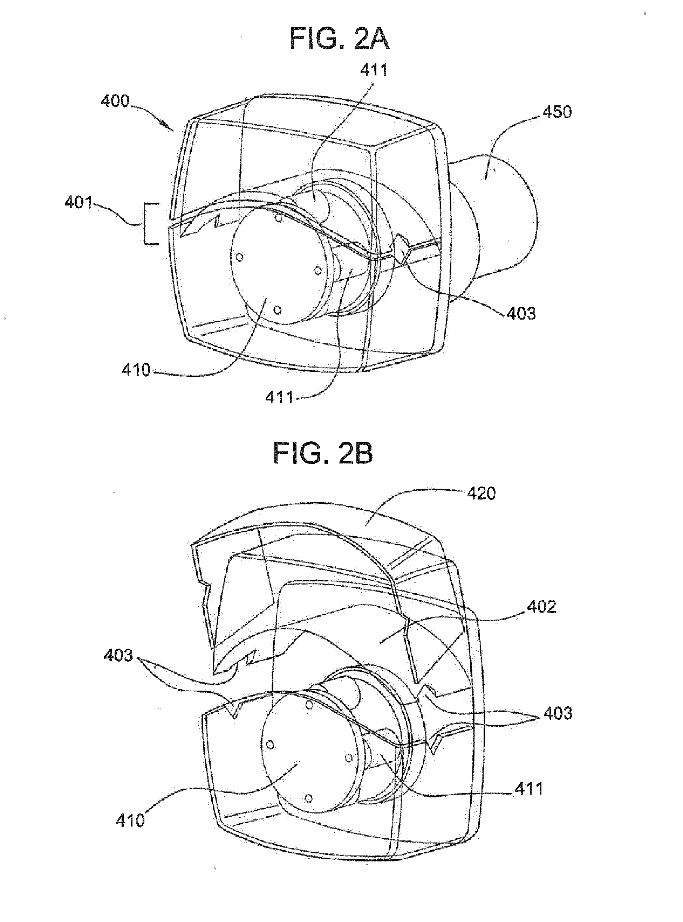

In accordance with the invention, and using Figures 1A, and 1B for general reference, the peristaltic pump 500, sometimes referred to as a roller pump, is a positive displacement pump, that moves fluid contained within a flexible tube. The pump includes a pump head 400 that includes (as shown in more detail in Figures 2A and 2B) a housing 401 and a rotatable rotor 410 including two or more (e.g., 2 to 12) shoes or rollers 411, typically attached to or near the external circumference of the rotor (the shoes or rollers can be integral with the rotor). The illustrated pump head includes a pinch block 402 and a tubing holder 403. If desired, the pump head housing can include a cover 420, e.g., for covering the rotor while the pump is operated. Typically, the pinch block and tubing holder can accommodate different diameter tubing (in the illustrated embodiment, cut-outs in the pinch block and the housing provide the tube holder, and other suitable arrangements for accommodating tubing are known in the art). A variety of pump heads are suitable for use in the invention, and are known in the art.

As shown in Figure 1A, the pump also includes a motor 450 (preferably, a non-reversible motor or a motor that can be programmed to be non-reversible) that rotates the rotor, and as the rotor turns, part of the tube is compressed by a shoe or roller, occluding the tube and forcing the fluid to move through the tube. After the shoe or roller passes, the tube opens, sometimes referred to as tube restitution or resilience. Since the rotor has a plurality of shoes or rollers occluding the tube as the rotor rotates (so that a portion of the tube is always occluded while the pump is operated), fluid flow occurs in one direction, and backward fluid flow is prevented. A variety of motors are suitable for use in the invention, and are known in the art.

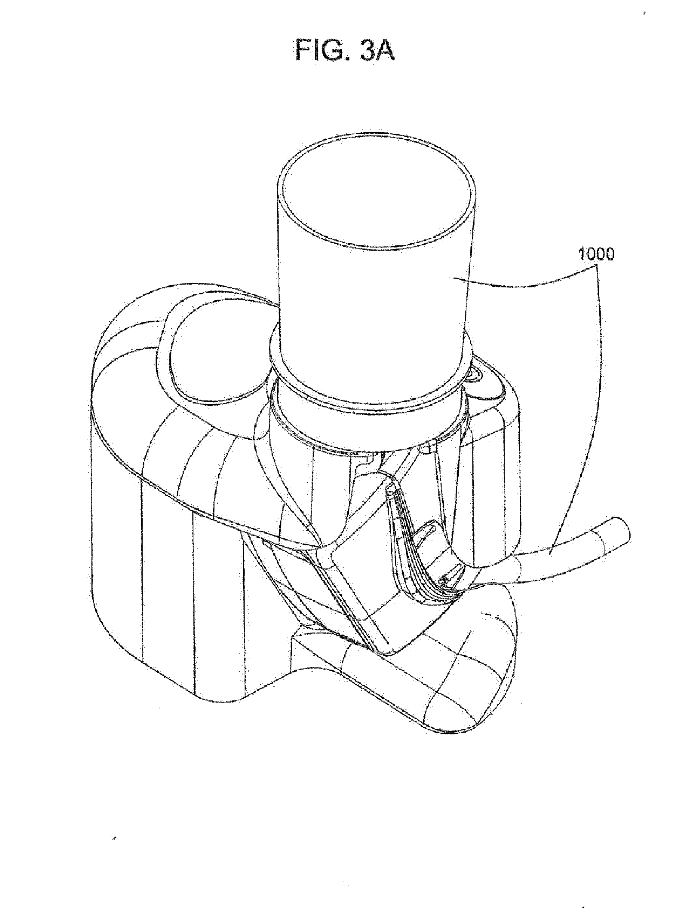

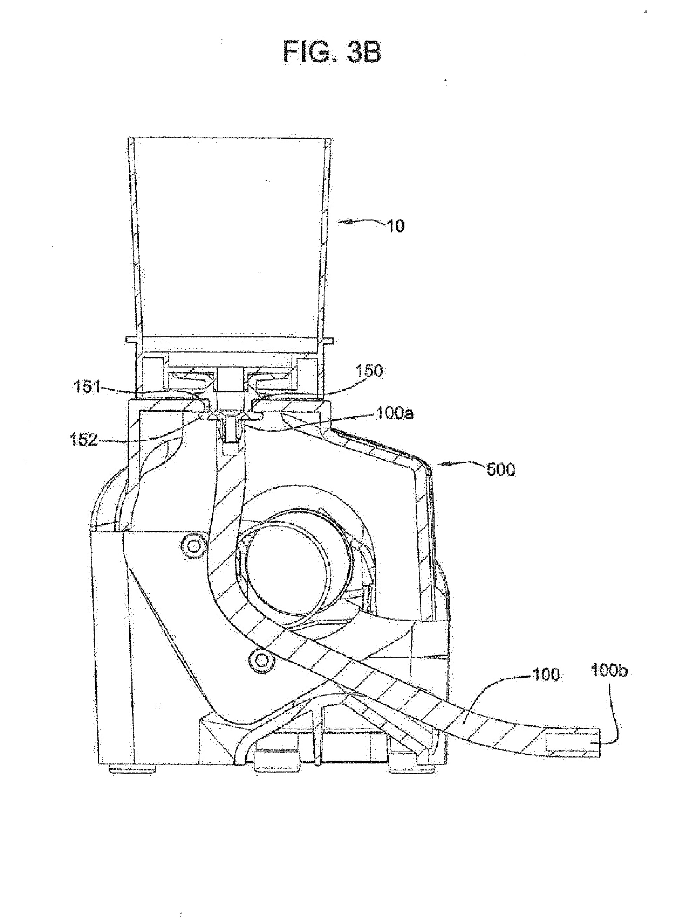

In accordance with the invention, and using Figures 3A and 3B for reference, the peristaltic pump 500 is located downstream of a filter assembly comprising a sample reservoir, a filter element, and a fluid port, wherein flexible tubing is attached to the fluid port, and the tubing is placed in the pump head 400, and the pump is arranged to pull a fluid sample potentially containing microbial contamination in a flow direction through the sample reservoir, the filter element, the fluid port, and the flexible tubing, without allowing fluid flow in the opposite direction.

Illustratively, as shown in Figures 1A and 1B, the pump 500 also includes a pump housing 200, preferably, for receiving the motor. Typically, the pump housing comprises a pump housing cover 210, the cover including (i) a substantially planar filtration assembly receiving portion 220 and (ii) a slot 230 in the receiving portion, wherein the slot includes a gap at least as large as the outer diameter of the fluid port of the filtration assembly, wherein the gap also is as large as the outer diameter of the flexible tubing without significantly compressing the tubing.

The pump can also include a power source for the motor (e.g., a battery or battery pack, that can be replaceable) and/or the pump can include a power cord for attaching the motor to a power source, e.g., an electrical outlet. Figure 1A illustrates an embodiment wherein the power source 550 comprises a battery pack.

Typically, the pump, that can be programmable, includes additional components such one or more of the following: switch(es), key pad(s), power indicator, battery charge indicator, circuit board(s), rotor speed indicator, motor control, rpm sensor. For some applications, a pump using a positional motor control and/or an rpm sensor can be particularly desirable for those applications wherein the exact fixed volume of fluid is to be measured and/or controlled for critical applications. Suitable pumps include also include programmable "smart pumps."

Suitable power sources, power cords, and additional pump components are known in the art.

Filtration assemblies suitable for use in the invention comprise a sample reservoir, a fluid flow port, and a filter element disposed in a fluid flow path between the sample reservoir and the fluid port so that the fluid to be filtered passes from the reservoir and through the filter element and fluid port. Typically, the assembly further comprises a base, e.g., supporting the sample reservoir, wherein the base comprises the fluid port. Alternatively, or additionally, the base can comprise a filter support surface for supporting the filter element and/or the assembly can further comprise an intermediate support member that is more porous than the filter element and which provides mechanical support to the filter element, such as a layer of mesh, screen, paper, or fabric. Preferably, the reservoir and base are detachably engaged, e.g., if it is desired to remove the filter element from the assembly after filtration.

Suitable filtration assemblies, that comprise a sample reservoir for holding the fluid to be filtered, a fluid flow port, and a filter element disposed in a fluid flow path between the sample reservoir and the fluid port so that the fluid to be filtered passes from the reservoir and through the filter element and fluid port, include, but are not limited to, those disclosed in U.S. Patents 4,468,321, 6,358,730 and 6,913,152, U.S. Patent Publication 2004/0063169, as well as those devices available from Pall Corporation (Port Washington, NY) as Magnetic Funnel Filters and devices available under the tradenames MicroFunnel^{TM} Filter Funnel, MicroFunnel™ Plus Filter Funnel, and MicroFunnel™ ST Disposable Filter Funnel. In accordance with the invention, flexible tubing is attached to the fluid port of a filtration assembly to provide a filtration assembly system.

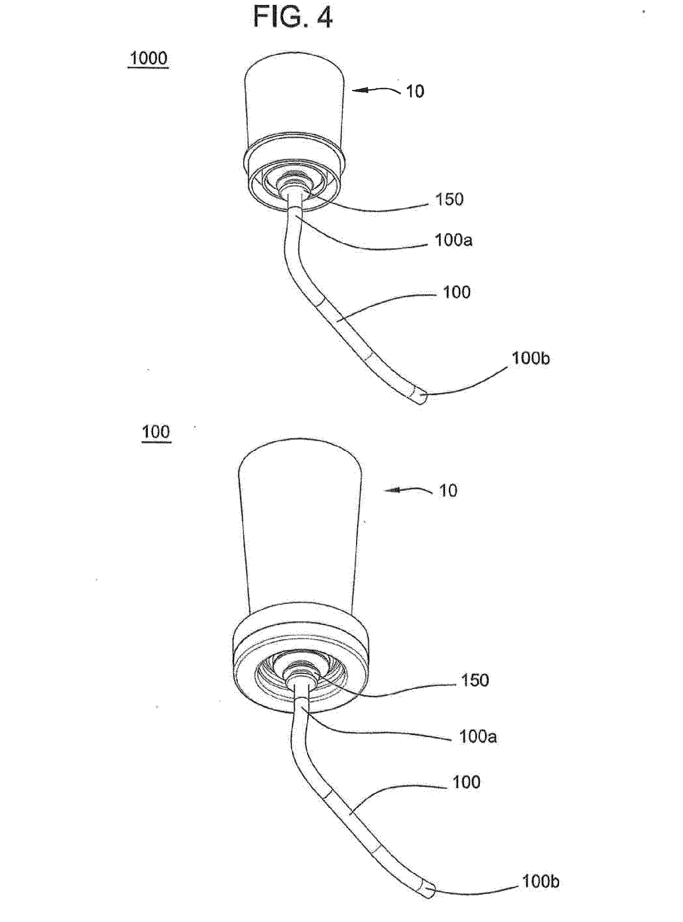

Illustrative embodiments of filtration assembly systems 1000 according to the present invention, each including a filtration assembly 10 communicating with flexible tubing 100, are illustrated in Figures 3A, 3B, and 4.

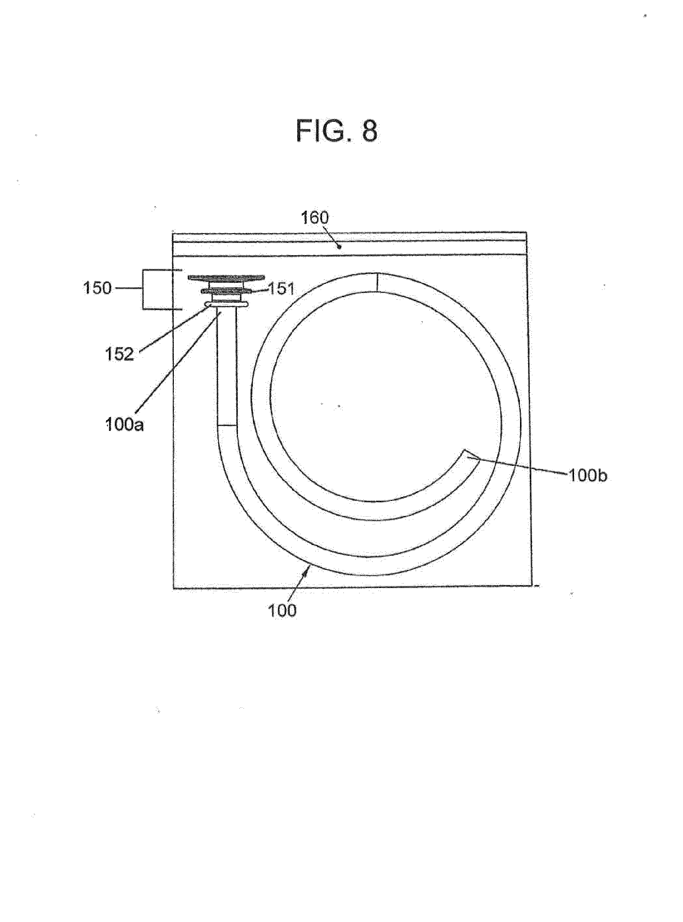

In some embodiments, the tubing is provided separately, and connected to the assembly before use. In one embodiment wherein the tubing is not integrally attached to the port of the filtration assembly, and the tubing is subsequently attached to the filtration assembly before use, one end of the tube further comprises, and attached thereto, an adapter such as a fitting that is fluid-tightly engageable with the port. Illustratively, as shown in Figures 3B, 4, and 8, a hollow fitting 150 is attached to one end 100a of the tubing 100 (the tubing 100 having ends 100a and 100b), and the fitting can be fluid-tightly engaged with the port 38 of the filtration assembly before use. Preferably, the fitting includes at least one outwardly facing projection, e.g., so that when a portion of the fitting is placed in the gap 230 of the pump housing 200, with the projection above (or below) the substantially planar filtration assembly receiving portion 220, the fitting is prevented from significant downward (or upward) movement. The exemplary fitting 150 shown in Figures 3B and 8, attached to one end 100a of tubing 100, comprises two axially spaced apart outwardly facing projections 151 and 152 arranged such that, when the fitting is placed in the gap 230, the projections prevent both significant downward and significant upward movement.

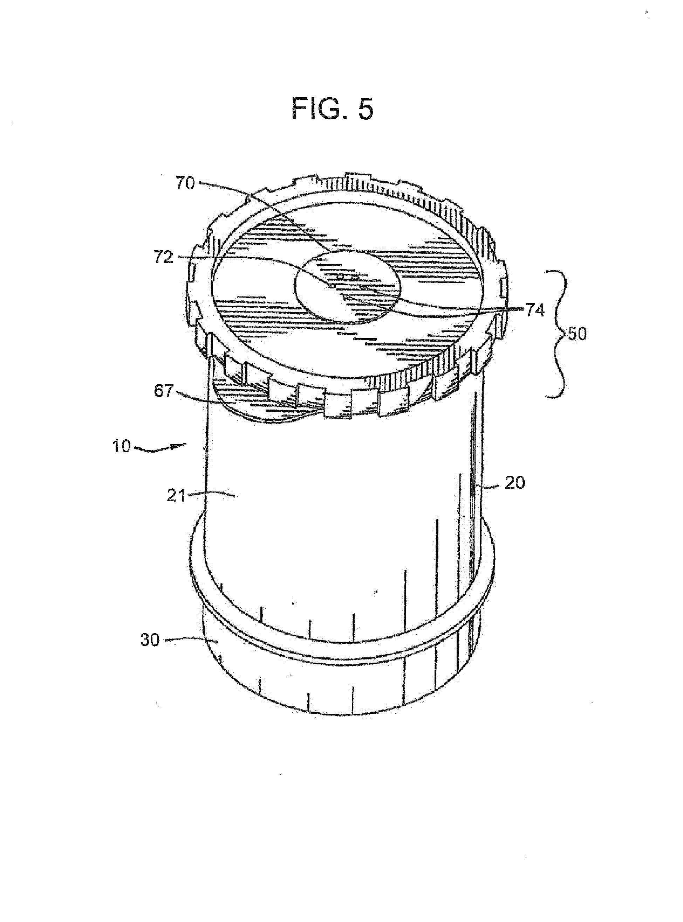

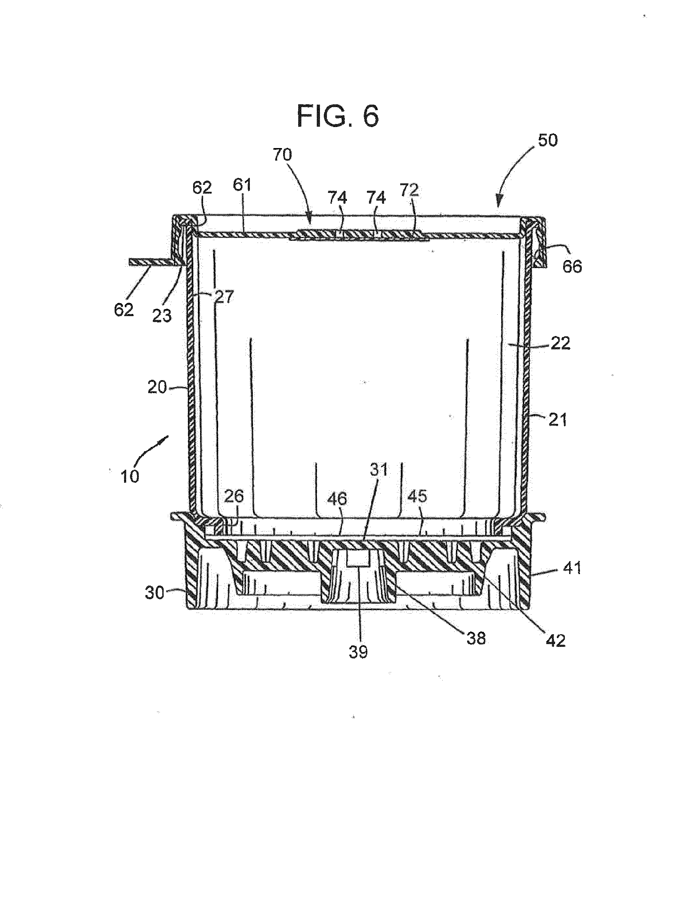

One exemplary filtration assembly 10 suitable for use in the invention is shown in more detail in Figures 5-7. The filtration assembly 10 shown in Figures 5 and 6 includes a sample reservoir 20 and a base 30 which may be detachably engageable with the lower end of the sample reservoir 20. The sample reservoir 20 defines a chamber 22 including an outer wall 21 and an inner wall 27, and the chamber 22 may be used to collect and hold a fluid sample (potentially containing microbial contaminants) which is to be filtered. In the illustrated assembly, the base 30 serves to support the sample reservoir 20 and includes a fluid port 38. A filter element 45 is disposed in a fluid flow path between the sample reservoir 20 and the fluid port 38 so that the fluid sample to be filtered passes from the reservoir, through the filter, the fluid port, and the flexible tubing 100. An optional cover member 50, when used, is preferably detachably engageable with the upper end of the sample reservoir 20 and forms a fluid-tight seal between the cover member 50 and the sample reservoir 20. In this illustrated assembly shown in Figures 5 and 6, the cover member 50 includes an optional vent 70 comprising a liquophobic element 72 which allows the passage of gas (e.g., air) but prevents the passage of liquid or microorganisms therethrough. However, the vent 70, if present, need not be associated with the cover member 50, it may, for example, be associated with a wall of the sample reservoir 20 (e.g., the outer wall 21, or the inner wall 27).

The sample reservoir 20 may have any structure which enables it to hold a desired volume of a sample fluid which is to be filtered. In the illustrated assembly, the sample reservoir 20 is generally cylindrical and is open at its upper and lower ends. The sample reservoir 20 has an outer wall 21 which defines the outer periphery of the sample reservoir 20 for the sample fluid, and an inner wall 27 that defines the inner periphery of the reservoir 20. The outer wall 21 may have a circular transverse cross-sectional shape and an inner diameter (provided by inner wall 27) which linearly decreases from its upper to its lower end. The shapes of the outer wall 21 and inner wall 27 are not critical and the diameter need not vary over its height. For example, the transverse cross-sectional shape may be polygonal or of a non-circular curved shape and the inner diameter or other dimensions of the sample reservoir 20 may be constant or vary in any desired manner over the height of the sample reservoir 20. The sample reservoir 20 may include gradations on its inner or outer surface to assist a user in measuring the amount of sample fluid being collected.

The illustrated filtration assembly 10 shown in Figures 5 and 6 includes a cover member 50, although a cover member is optional. One example of the cover 50, which is best illustrated in Figure 6, is shaped so as to detachably fit atop the upper end of the sample reservoir 20. The cover 50 may engage with the upper end of the sample reservoir 20 in various manners. For example, they may engage each other with a snap fit, a bayonet fit, threaded engagement, press fit, or a loose fit. Preferably, however, the engagement provides a fluid-tight seal between the cover and the reservoir, and the engagement is such as to provide some resistance to disengagement of the cover 50 from the sample reservoir 20 so as to enable the filtration assembly 10 to be handled and transported without the cover 50 falling off the sample reservoir 20, while still permitting the cover 50 to be readily detached from the sample reservoir 20. Additionally, the cover member 50 may be shaped so as to be detachably connectable to the base 30, for example, when the cover member 50 and base 30 together are to be used as part of a petri dish.

While including a vent is optional, the illustrated filtration assembly 10 shown in Figures 5 and 6 includes at least one vent 70 comprising at least one, and preferably a plurality of, ports 74, and a liquophobic element 72. Including a vent allows the fluid to be filtered to be withdrawn from the filtration assembly 10 without removing the cover 50.

In the illustrated assembly shown in Figures 5 and 6 , the cover 50 includes the optional vent 70, and the vent includes five ports 74. Liquophobic element 72 disposed in the flow path through the vent 70, which prevents the passage of possible external environmental contaminants, e.g., microorganisms, liquids, dust, etc., into the sample reservoir 20, but allows the passage of air through the port(s) 74 into the sample reservoir 20 after the sample is collected and the cover 50 is fluid tightly engaged with the reservoir 20. Element 72 can be attached to the filtration assembly, e.g., to cover 50 and/or a wall of the sample reservoir 20, as is known in the art.

Typically, the liquophobic element 72 has a pore structure, e.g., a pore size (for example, as evidenced by bubble point, or by K_{L} as described in, for example, U.S. Patent No. 4,340,479), a pore diameter, a pore rating, or a fine particle arresting efficiency (as described in, for example, the Monodisperse Dioctyl Phthalate (DOP) Smoke Test (ASTM D2986-71, or ASTM D 2986-95a)) that reduces the passage therethrough of microorganisms, preferably reducing the passage of bacteria therethrough. For example, in some embodiments, the liquophobic element 72 has a pore size of about 2.0 micrometers or less, or about 1.0 micrometer or less. Alternatively, in some embodiments, the liquophobic element 72 removes at least about 99.9% of particles having diameters of at least about 0.2 microns (µm) or greater, or the element removes at least about 99.97% of particles having diameters of at least about 0.3 µm or greater. Illustratively, in one embodiment, the liquophobic element has a Monodisperse Dioctyl Phthalate (DOP) Smoke Penetration (e.g., as measured by ASTM D2986-71) at 0.3 µm at 10.5 ft/minute gas flow of at least about 0.03%.

The liquophobic element can be of any desired type, such as a microporous membrane (e.g., commercially available from Pall Life Sciences (Ann Arbor, MI) and Pall Corporation (East Hills, NY)), or a porous plastic member, e.g., POREX^{®} porous plastic components commercially available from Porex Corporation (Fairburn, GA). Preferably, the liquophobic element comprises a hydrophobic microporous membrane. Suitable liquophobic elements including elements having a bacterial blocking pore structure are commercially available.

Although in the illustrated assembly, the vent 70 is located on the surface of cover 50 facing the interior of the sample reservoir 20, the optional vent may be disposed in other locations. For example, the vent can be disposed in or on the outer wall 21 or the inner wall 27 of the sample reservoir 20, or the vent can be in or on other portions of the cover. Additionally, the vent can include any suitable number of ports, and the ports can have any suitable inner diameters.

Prior to use, the vent 70 may be covered with a removable element, for example, to prevent exposing the element 72 to undesirable material (e.g., sterilizing agents) before use. The vent 70 may be covered using any suitable material including, for example, a sticker, label or seal and/or an additional cover member. A sticker covering the vent 70 preferably utilizes an adhesive providing some resistance to removal but still allowing easy removal. The sticker may be sized to cover only the vent 70 or alternatively may extend beyond the area of the vent. The sticker may contain pre-printed indicia and/or may comprise a material upon which a user may mark or write. Additionally or alternatively, an additional cover member may engage with the cover 50 in various manners and with varying degrees of tightness as described above with respect to the sample reservoir 20 and the base 30 and the cover 50. The additional cover member may also be shaped so as to fit atop the upper end of the base 30, thereby enabling the additional cover and the base 30 together to form a petri dish.

In the illustrated assembly shown in Figures 5 and 6, the cover 50 that includes vent 70 comprises a disc-shaped plate 61 including a tab 67 protruding from the outer periphery of the plate 61. The cover 50 includes a continuous annular inverted channel 62 formed around the entire outer periphery of the disc-shaped plate 61. As shown in more detail in Figure 6, the inverted channel 62 preferably comprises an inner wall extending upwards from the upper surface of the disc-shaped plate 61, a generally right angular ledge around the top edge of inner wall and extending outwardly from the center of the disc-shaped plate 61 and an outer wall extending downwardly from the right annular ledge. Using the assembly shown in Figure 6 for reference, a snap-fit is formed between the inverted channel 62 and a radially outward lip 23 formed around the entire outer periphery of the upper end of the sample reservoir 20. The outer wall has a radially inward bulge 66. The outer diameter of the inverted channel 62 measured at the bulge 66 in a relaxed (unstressed) state is smaller than the outer diameter of the sample reservoir 20 at the lip 23 in a relaxed state so that once the lip 23 is urged upwards past the bulge 66, the bulge 66 will resist disengagement of the sample reservoir 20 and the cover 50. The engagement between the cover 50 and the sample reservoir 20 may be of varying degrees of tightness. For example, the engagement may be sufficient to provide some resistance to disengagement without forming a seal, or preferably, the engagement provides a fluid-tight seal between the two members.

As noted above, a cover is optional. However, a fluid-tight seal between the cover 50 and the sample reservoir 20 is convenient when the sample reservoir 20 is to be used for collecting and/or temporary storage of a fluid sample prior to filtration. For example, in factories and fluid processing facilities, it is common to collect a fluid sample in one part of the factory or facility and then to carry the sample to a laboratory for analysis in a different part of the factory or facility. In such cases, the provision of a fluid-tight seal between the cover 50 and the sample reservoir 20 (and the configuration of the vent) enables a fluid sample within the sample reservoir 20 to be transported from one location to another without fear of spilling or contamination. The provision of tab 67 facilitates disengagement of the cover 50 from the reservoir 20, for example, to introduce the sample into the sample reservoir. A fluid-tight seal can be formed by any suitable means, but preferably by one which does not require the use of a separate sealing member, such as an O-ring or a gasket. In the illustrated embodiment, a fluid-tight seal is achieved between the cover 50 and the sample reservoir 20 by the inverted channel 62. The inner diameter of the inverted channel 62 in a relaxed state is larger than the inner diameter of the upper end of the sample reservoir 20 in a relaxed state so that when the lip 23 of the sample reservoir 20 is placed into the inverted channel 62, the upper end of the sample reservoir 20 will be urged radially outwards by the inner wall of the inverted channel 62 towards the outer wall of the inverted channel 62. The upper end of the sample reservoir 20 is thereby pressed into intimate contact with the inverted channel 62, resulting in the formation of a fluid-tight seal between the cover 50 and the sample reservoir 20 around the entire periphery of the sample reservoir 20.

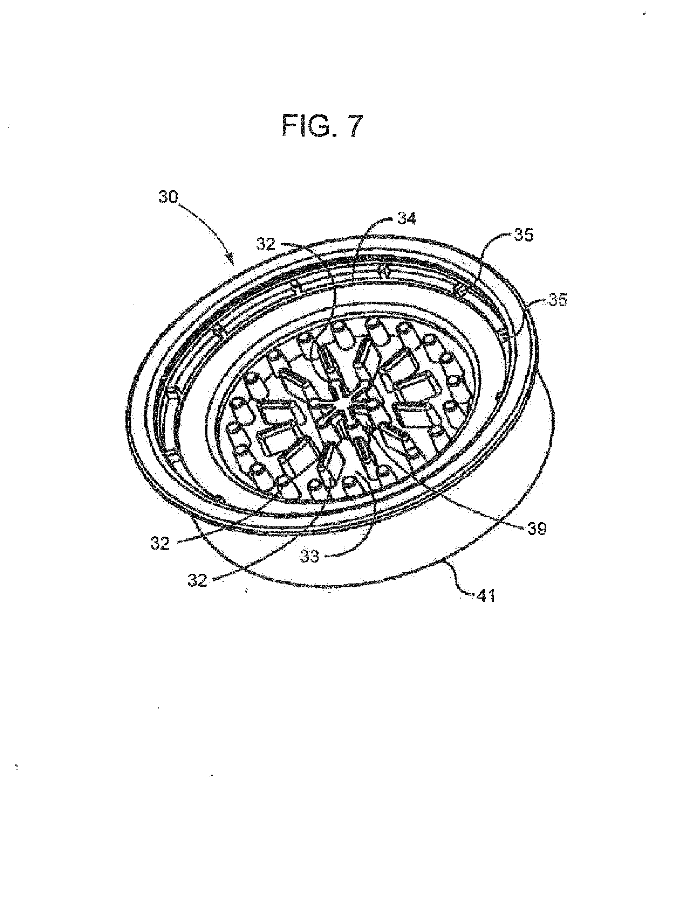

In the illustrated filtration assembly, the assembly includes a base 30. The base 30 preferably provides support for the sample reservoir 20. The base 30 may also include a fluid port and/or a filter support surface 31. As illustrated in Figure 7, which is a top isometric view of an illustrative base 30, the illustrated base 30 includes a filter support surface 31 and a fluid port 38. The illustrated filter support surface 31 is defined by the upper surfaces of a plurality of projections 32 which extend upwards from a bottom inner surface 33 of the base 30. The projections 32 are spaced from each other to enable filtrate which has passed through the filter element 45 to flow between the projections 32 and out through the fluid port 38. One or more drainage openings 39 for filtrate are formed in the projections 32 at the center of the base 30 to connect the interior of the fluid port 38 with the region of the base containing the projections 32.

In the illustrated assembly, the base 30 is a unitary member formed by injection molding, for example, with the filter support surface 31 being integrally formed with other portions of the base 30. However, it is also possible for the base 30 to comprise a plurality of separately formed components. For example, the filter support surface 31 may comprise a perforated plate, a porous plate, a screen, or a mesh which is removably installed within the interior of the base 30 and has an upper surface which can support the filter element 45.

The filter support surface 31 as illustrated is planar, but it may have any shape which enables it to support the filter element 45 for filtration. For example, the filter support surface 31 may be dished, arched or wave-like in shape.

In the illustrated assembly, the filter support surface 31 is surrounded by a circular wall 34 extending upwards from the outer periphery of the filter support surface 31, and a plurality of radial projections 35 extend upwards from a ledge formed atop the wall 34, with the vertical radially inner surface of each projection 35 being flush with the wall 34. The wall 34 and the projections 35 serve to surround and position a filter element 45 disposed on the filter support surface 31.

Although the filter support surface 31 is shown as a component of the base 30, the support surface is not so limited. The filter support surface 31 may be formed or positioned within the sample reservoir 20. For example, instead of the sample reservoir 20 being completely open at its lower end, it may have a perforated bottom surface for supporting a filter element 45. Alternatively, the filter support surface 31 may be omitted entirely, for example, if the filter is self-supporting.

The filtration assembly 10 can be capable of standing upright on a level surface without being supported when the tubing is not attached. In one illustration, the base 30 includes an outer wall 41 extending around its entire periphery for supporting the base 30 on a table or other level surface. However, members other than a continuous wall can also be used to support the base, such as a plurality of legs. Alternatively, the base 30 may not be self-supporting, and it may have a shape which does not stand upright by itself. For example, the bottom of the base 30 may be shaped like a funnel.

The sample reservoir 20 and the base 30 may have a variety of configurations. In one embodiment, the sample reservoir 20 and the base 30 may be separately formed and permanently connected to each other, or they may be formed as a single member. In another example, the filtration assembly 10 may not include a base 30. However, in the illustration, the sample reservoir 20 is detachably engaged with the base 30 so that the base 30 can be separated from the sample reservoir 20, for example, to remove the filter element 45 or so that the base 30 can be used separately from the sample reservoir 20 as part of a petri dish.

The manner of engagement between the sample reservoir 20 and the base 30 is preferably such that the engagement creates a fluid-tight seal without the need for a sealing member, such as an O-ring or gasket, yet such that the sample reservoir 20 and the base 30 can be readily disengaged from each other by hand. The lower end of the sample reservoir 20 is also preferably shaped so that a fluid-tight seal is formed between the sample reservoir 20 and the upper surface of a filter element 45 disposed on the filter support surface 31 to prevent fluid from the sample reservoir 20 from bypassing the filter element 45 by flowing between the sample reservoir 20 and the filter element 45.

Advantageously, any type of detachable engagement providing intimate, sealing contact between the sample reservoir 20 and the base 30 around the entire inner periphery of the base 30 may be employed to detachably engage the two members. For example, there may be an interference fit between the sample reservoir 20 and the base 30 so that a radial force presses a peripheral surface of the sample reservoir 20 into sealing contact with an opposing peripheral surface of the base 30, or opposing surfaces of the sample reservoir 20 and the base 30 may be pressed into sealing contact with each other by a compressive force acting in the axial direction of the filtration assembly 10, and/or the reservoir and base can each include magnets wherein the magnets are adjacent each other when the reservoir and base are assembled wherein magnetic attraction sealingly clamps the filter element between the reservoir and base. In the illustrated assembly, the sample reservoir 20 and the base 30 are engaged with each other by an interference fit which produces a fluid-tight seal between the outer peripheral surface of the sample reservoir 20 and the inner peripheral surface of the base 30. The sample reservoir 20 and the base 30 may be structured so as to provide resistance to an axial force tending to pull them apart so as not to be inadvertently disconnected from each other during use.

In the present illustration, resistance to disengagement is provided by a snap fit in which the lower end of the sample reservoir 20 is received inside the upper end of the base 30. As shown in the cross-sectional elevation of Figure 6, the lower end of the sample reservoir 20 has a groove and a radially outward projection which extend continuously around its entire outer periphery. Similarly, the illustrated base 30 has a groove and a radial inward projection extending continuously around its entire inner periphery at its upper end. The outer diameter of the lower end of the sample reservoir 20 and the inner diameter of the base 30 are preferably selected so that the projections will snap into and fit snugly inside the respective grooves, with an interference fit so that there is intimate contact, such as line contact or surface contact, between each projection and the corresponding groove around the entire circumference of the sample reservoir 20. The sample reservoir 20 may be disconnected from the base 30 simply by flexing the two members with respect to each other, for example, to disengage the projections from the grooves.

It is generally easier to disengage the two members if the groove and the projection are formed as close to the upper end of the base 30 as possible. For example, in the illustrated embodiment, projection immediately adjoins the upper end of the base 30. The location of the sealing contact between the sample reservoir 20 and the base 30 is not critical so long as the contact can prevent fluid from leaking to the exterior of the filtration assembly 10 during normal use. For example, the sealing contact may be between the mating surfaces of the grooves and the projections, or it could be formed in a different location, with engagement between the grooves and the projections serving primarily to resist inadvertent disengagement of the sample reservoir 20 and the base 30 or to maintain an axial compressive force between the sample reservoir 20 and the filter element 45 to form a fluid-tight seal against the filter element 45. In the latter case, the grooves and the projections need not be continuous members.

In the illustrated assembly, each groove is complementary in shape with the corresponding projection, i.e., it has substantially the same radius of curvature as the corresponding proj ection so that each groove and the corresponding proj ection are in surface contact, but the curvatures of the groove and the projection may alternatively be such that they are in line contact, for example. It is possible to form a seal between the sample reservoir 20 and the base 30 with a single projection formed on the surface of one of the two members and a single groove for engagement with the projection formed on the surface of the other two members, but a plurality of grooves and projections may create a seal of greater integrity.

Many other arrangements besides a snap fit can be used to resist disengagement between the sample reservoir 20 and the base 30, such as a bayonet fit or threaded engagement, or via magnets as discussed above. It may also be desirable to dispose tape or a sleeve, such as a shrink wrap sleeve, around the joint between the sample reservoir 20 and the base 30 or to lightly weld or bond the two members to each other (such as by ultrasonic welding) around their peripheries to secure the members together while enabling them to be easily disconnected from each other when desired. Such a manner of connection may be employed instead of or in addition to the interference fit provided by the grooves and projections on the sample reservoir 20 and the base 30.

The lower end of the sample reservoir 20 can be formed with an annular sealing rim 26 which extends around the entire periphery of the sample reservoir 20. When the grooves and the projections of the sample reservoir 20 and the base 30 are engaged with each other, the sealing rim 26 is pressed downwards into sealing contact with the upper surface of the filter element 45 disposed atop the filter support surface 31 of the base 30. The compressive force between the sealing rim 26 and the filter element 45 is maintained by the engagement between the grooves and the projections of the sample reservoir 20 and the base 30. In the illustrated assembly, the sealing rim 26 is positioned on the sample reservoir 20 such that an annular air space is present between the outer periphery of the sealing rim 26 and the inner periphery of the base 30 around the entire circumference of the sealing rim 26. It is thought that the air space may improve the integrity of the seal between the sample reservoir 20 and the base 30 by forming an air lock which prevents creeping of fluid by capillary action between the two members. However, the air space is not essential, and the sealing rim 26 may closely contact the inner periphery of the base 30.

A filter element 45 preferably comprises at least one filter medium compatible with the fluid being filtered and capable of removing microorganisms of interest from the fluid. The filter medium may be of any desired type, such as a microporous membrane or fibrous element of various materials, or filter paper, for example. For some applications, the filter medium is a thermally resistant material. A wide variety of filter media for microbiological studies are commercially available, and any such filter media can be employed with the present invention as the filter element 45. The filter medium may collect (capture) microorganisms in any desired manner, e.g., according to size, by adsorption, and/or affinity binding. Filter media for use in microbiological studies are frequently flat membrane discs, but the filter element 45 need not have any particular shape. For example, instead of being flat, the membrane may include pleats to increase its surface area.

The filter element 45 is disposed in a fluid flow path between the sample reservoir 20 and the fluid port 38 so that the fluid to be filtered passes through the filter element 45. The filter element may be self-supporting or alternatively as in the illustrated embodiment, the flat filter element 45 is supported by a filter support surface 31. The filter element 45 may be removably supported by the filter support surface 31 or may alternatively be permanently affixed to the filter support surface 31, for example by using an adhesive, a solvent, radio frequency sealing, ultrasonic sealing and/or heat sealing. Although in the illustrated assembly, the base includes the filter support surface 31, alternatively the sample reservoir 20 may include a filter support surface. The filter element 45 may directly contact the filter support surface 31 or it may preferably rest upon an intermediate support member, which is more porous than the filter element 45 and which provides mechanical support to the filter element 45, such as a layer of mesh, paper or fabric. Alternatively, the filter element can comprise a filter medium laminated to a support. If the filter element 45 is to be left on the base 30 during incubation, it may be convenient if an absorbent pad 46 for use in holding a nutrient solution during incubation is placed beneath the filter element 45 prior to filtration rather than afterwards to reduce the amount of handling of the filter element 45 after filtration. Furthermore, the absorbent pad 46 may provide support for the filter element during filtration. It may also be desirable to place a prefilter, a protective sheet or other member atop the filter element 45.

It may be advantageous to place a resilient, compressible member between the lower surface of the filter element 45 and the filter support surface 31 in the region beneath where the sealing rim 26 contacts the filter element 45. Such a member can compensate for variations in the axial length of the sealing rim 26 and the filter support surface 31 to maintain the sealing rim 26 in intimate, sealing contact with the filter element 45, thereby enabling the manufacturing tolerances of the sample reservoir 20 and the base 30 to be less precise. The resilient member may be either permeable or impermeable to the fluid being filtered. For example, it may comprise an impermeable gasket disposed beneath the filter element 45. It is also possible to place a resilient sealing member, such as a gasket, between the top surface of the filter element 45 and the sealing rim 26 so that the sealing rim 26 does not directly contact the filter element 45 but is pressed into sealing contact with the sealing member, which in turn is pressed into sealing contact with the filter element 45. Such a sealing member may be separate from or joined to the filter element 45.

In the illustrated assembly, the wall 34 surrounding the filter support surface 31 has a height such that when an absorbent pad 46 and a filter element 45 are mounted on the filter support surface 31, the absorbent pad 46 will be surrounded by the wall 34 and disposed at least partially below the upper end of the wall 34, while the filter element 45 disposed atop the absorbent pad 46 will be positioned at or above the upper end of the wall 34 and will be surrounded by the radial projections 35. For example, the wall 34 may have a height substantially the same as the thickness of the absorbent pad 46. With the absorbent pad 46 located partially or entirely below the upper end of the wall 34, when a user of the filtration assembly system 1000 comprising the filtration assembly 10 wishes to transfer the filter element 45 from atop the absorbent pad 46 to a different location, it is easy for the user to pick up the filter element 45 using forceps without picking up the absorbent pad 46 as well. The spaces between the radial projections 35 provide easy access to the filter element 45 and facilitate its removal from the base 30.

From the standpoint of ease of manufacture, it is preferable if the axial length of the sealing rim 26 of the sample reservoir 20 and the axial height of the radial projections 35 on the base 30 are such that when the sample reservoir 20 sealingly engages the base 30 and the sealing rim 26 of the sample reservoir 20 is pressed into sealing contact with the filter element 45 as shown in Figure 6, there is an axial gap between the top surface of the radial projections 35 and the bottom surface of the sample reservoir 20. If such a gap is present, the radial projections 35 and the sealing rim 26 do not need to be manufactured to as precise tolerances as when the upper surfaces of the radial projections 35 contact the bottom surface of the sample reservoir 20.

The filtration assembly system 1000 and filtration assembly 10 can be made from a wide variety of materials, including those conventionally used for funnels, reservoirs, petri dishes, tubing and other laboratory equipment, such as metals, plastics, and glass, depending upon factors such as the desired strength, flexibility, heat resistance, and corrosion resistance and upon whether the filtration assembly 10 is intended to be reusable or discarded at the completion of use. Different portions of the filtration assembly system 1000 and assembly 10 may be formed of different materials. For economy of manufacture, plastics which can be shaped by molding are particularly suitable for the filtration assembly 10. Some examples of suitable plastics are polypropylene, nylon, and polyacrylate. In some instances, it is convenient if portions of the assembly 10, such as the sample reservoir 20 are translucent or transparent to permit substances within the assembly 10 to be readily observed.

Typically, the filtration assemblies are shipped in a sealed container, such as a bag, while maintaining sterility. The sealed container can also include tubing, either attached to the fluid port (providing a filtration assembly system), or unattached, preferably while maintaining the sterility of the filtration assembly and the tubing. Alternatively, the tubing (preferably, attached to an adapter comprising a fitting) can be shipped in a separate sealed container (e.g., sealed bag 160, shown in Figure 8), typically while maintaining sterility. The filtration assembly, adapters, tubing, and filtration assembly system, can be sterilized in accordance with a variety of sterilization protocols known in the art.

In some embodiments, the first and/or second end of the tubing is sealed before use, e.g., wherein either or both ends are covered by a removable seal, or either or both ends are closed and the tubing is cut to open either or both ends, or one end is covered by a removable seal, and the tubing is cut to open the other end.

While the tubing 100 contacted by the shoes or rollers of the peristaltic pump is flexible, embodiments of the invention can include additional tubing, wherein the additional tubing is flexible or non-flexible. A variety of flexible and non-flexible tubing, as well as adapters, fittings, and connectors, can be used in accordance with the invention, and suitable tubing, adapters, fittings, and connectors are known in the art.

A method for processing a fluid potentially containing microbial contaminants according to an embodiment of the invention comprises operating a peristaltic pump downstream of, and in fluid communication via flexible tubing with, a filtration assembly comprising a sample reservoir, a microbe-collecting filter element, and a fluid port, the reservoir containing fluid potentially containing microbial contaminants, wherein operating the pump comprises compressing and opening the flexible tubing and pulling the fluid in a flow direction through the sample reservoir, the filter element, the fluid port, and the flexible tubing, without allowing fluid flow in the opposite direction.

Embodiments of the method can further comprise analyzing the filter element for the presence of microbes and/or analyzing the fluid that passed through the filter element for the presence of microbes.

Typically, an embodiment of the method further comprises directing the fluid passing through the tubing to a desired location.

A variety of fluids can be filtered in accordance with embodiments of the invention, e.g., fluids in the biopharmaceutical, microelectronics, and beverage industries. Embodiments of the invention are particularly useful where it is desirable to monitor the contamination of the fluid e.g., to ensure that the fluid is sterile. Embodiments of the invention are suitable for use in a variety of systems, including "hot loop" systems, e.g., wherein the fluid to be filtered is a heated fluid, e.g., heated to a temperature of about 80°C.

In carrying out an embodiment of the method, and using Figures 3A and 3B for general reference, the filtration assembly system 1000, comprising the filtration assembly 10, and tubing 100 communicating with the fluid port 38, is associated with the peristaltic pump 500, wherein a portion of the tubing is placed in the pump head (e.g., placed in the tubing holder 403 and arranged between the rotor 410 and the pinch block 402; see Figure 2B), the portion of the tubing near the port 38 is placed in the slot 230, and the base of the assembly is placed on the planar assembly-receiving portion 220 of the pump housing 200. As noted above, the filtration assembly may include integrally-attached tubing, or the tubing can be subsequently attached, e.g., by engaging fitting 150 with port 38.

Preferably, the fluid sample to be filtered is originally collected in the sample reservoir 20, alternatively, the fluid may be placed in the sample reservoir after collection, and either before or after the assembly 10 is placed on the pump housing. Embodiments of the method can be carried out with filter assemblies having a cover, or without a cover. Also, as noted above, the filter assembly can include a one or more vents (e.g., as part of the cover and/or as part of another component of the assembly), or the assembly can lack a vent.

With the filtration assembly 10 on the pump housing 200 (and if a cover is used, cover 50 can be fluid-tightly engaged with the reservoir 20), the peristaltic pump 500 is operated to pull the fluid sample through the filter element 45 and the port 38 into the tubing 100. The filtered fluid can be passed to the desired location.

When the fluid sample has been pulled out of the sample reservoir 20 and through the filter element 45, the pump is typically turned off. At this time, the filtration assembly 10 may be removed from or left on the pump housing. Typically, the assembly and tubing are removed from the pump and discarded when appropriate.

The filtered fluid and/or the filter element can be tested for the presence of microbes, as is known in the art.

With respect to testing the filter element, the filter element can be removed from the filter assembly and transferred to, for example, a petri dish, wherein the captured microorganisms, if present, can be cultured. Testing the filter element, including culturing the microorganisms, and determining the presence and/or identity of the microorganisms, can be carried out as is known in the art.

In those embodiments wherein the base 30 and cover 50 or base 30 and additional cover are to be used as a petri dish, after the completion of filtration, the sample reservoir 20 is detached by hand from the base 30 by releasing the snap fit between them, and the filter element 45 (that can be removable, or permanently affixed to the filter support surface 31) is left atop the base 30 where a suitable nutrient solution is applied to the absorbent pad 46 located beneath the filter element 45, the absorbent pad 46 typically having been placed beneath the filter element 45 prior to filtration.

The nutrient solution can be applied to the absorbent pad 46, either from above through the filter element 45 or from below via the fluid port 38. Once the nutrient solution has been applied to the absorbent pad 46, the petri dish comprising the base 30 and the cover 50 or the base 30 and additional cover member are ready to be incubated. If desired, a closure, such as a cap or a plug, may be mounted on the lower end of the fluid port 38, or inserted an open end of the tubing distal to the port, to prevent fluid from leaking out of it during incubation.

In accordance with an embodiment of the invention, a test kit is provided, comprising the filtration assembly system, and one or more of a nutrient solution, a growth medium, and a reagent (e.g., for detecting the presence of the microorganism(s)). Preferably, the test kit includes a sterile filtration assembly sealed in one container, while the nutrient solution, growth medium and/or reagent(s) are sealed in another container.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A filtration assembly system for use with a peristaltic pump comprising:
a filtration assembly, the assembly comprising
(a) a sample reservoir for holding a fluid sample to be filtered;
(b) a fluid port in fluid communication with the sample reservoir;
(c) a filter element disposed in a flow path between the sample reservoir and the fluid port;
(d) a filter support surface for supporting the filter element; wherein the system further comprises
(e) a flexible tube having a first end and a second end, wherein the first end is connectable to the fluid port to provide fluid communication between the first end and the fluid port, or the first end is connected to the fluid port and the first end is in fluid communication with the port.

2. The system of claim 1, wherein the first end of the tube is connected to the fluid port, optionally wherein the first end of the tube is integrally connected to the fluid port.

3. The system of claim 2, wherein the first end of the tube further comprises a fitting fluid-tightly engageable with the port.

4. The system of any one of claims 1 to 3, wherein the fitting further comprises at least one outwardly facing projection, optionally wherein the fitting comprises at least two axially spaced apart outwardly facing projections.

5. The system of any one of claims 1 to 4, wherein the filtration assembly further comprises a base, the base supporting the sample reservoir, optionally wherein the base comprises the fluid port.

6. The system of claim 5, wherein the base comprises the filter support surface for supporting the filter element.

7. The system of any one of claims 1 to 6, further comprising a cover member detachably and replaceably covering the sample reservoir with a fluid-tight fit.

8. The system of any one of claims 1 to 7, further comprising at least one vent in communication with the sample reservoir, wherein the vent includes a liquophobic element that allows air into the sample reservoir but prevents the passage of microorganisms into the sample reservoir.

9. A peristaltic pump for use with a filtration assembly system comprising
(a) a peristaltic pump head, the head comprising a head housing, a rotatable rotor comprising at least two shoes or rollers, and a tubing holder suitable for receiving flexible tubing, the flexible tubing having an outside diameter, wherein the shoes or rollers are arranged to compress the flexible tubing placed in the holder, and wherein the flexible tubing is in fluid communication with a fluid port of a disposable filtration assembly; the fluid port, having an outside diameter, also being in fluid communication with a sample reservoir of the disposable filtration assembly, the fluid port being arranged to receive filtered liquid passing through a filter element in the filtration assembly;
(b) a motor communicating with the pump head, arranged to rotate the rotor;
(c) a pump housing receiving the motor, the pump housing comprising a pump housing cover, the cover including (i) a substantially planar filtration assembly receiving portion and (ii) a slot in the receiving portion, wherein the slot includes a gap at least as large as the outer diameter of the fluid port of the disposable filtration assembly, wherein the gap also is as large as the outer diameter of the flexible tubing without significantly compressing the tubing;
wherein the pump is arranged to pull fluid in a flow direction through the sample reservoir, the filter element, the fluid port, and the flexible tubing, without allowing fluid flow in the opposite direction.

10. The pump of claim 9, wherein the slot is arranged to receive a portion of a fitting engageable with the fluid port, the fitting having one end of the flexible tubing attached thereto.

11. The pump of claim 9 or 10, comprising a non-reversible motor.

12. The pump of claim 9 or 10, wherein the pump is programmed to operate the motor non-reversibly.

13. A method for processing a fluid potentially containing microbial contaminants comprising:
operating a peristaltic pump downstream of, and in fluid communication via flexible tubing with, a filtration assembly comprising a sample reservoir, a microbe-collecting filter element, and a fluid port, the sample reservoir containing the fluid potentially containing microbial contaminants; wherein operating the pump comprises compressing and opening the flexible tubing and pulling the fluid in a flow direction through the sample reservoir, the filter element, the fluid port, and the flexible tubing, without allowing fluid flow in the opposite direction.

14. The method of claim 13, further comprising analyzing the filter element for the presence of microbes.

15. The method of claim 13 or 14, further comprising analyzing the fluid for the presence of microbes.
